# FASCICULE DE BREVET EUROPEEN

(11) **EP 1 302 447 B1**
(45) Date de publication et mention de la délivrance du brevet: **03.04.2013**
(21) Numéro de dépôt: 02292409.6
(22) Date de dépôt: 01.10.2002
(51) Int. Cl.: C02F 3/34, C12R 1/32

(54) **Procédé de traitement d'effluents aqueux contenant du methyl-tert-butyl ether et/ou du methyl-tert-amyl ether par Mycobacterium austroafricanum CNCM I-2562**
Verfahren zur Behandlung von Methyl-tert-Butylether und/oder tert-Amylmethylether enthaltendem Abwasser durch Mycobacterium austroafricanum CNCM I-2562
Process for the treatment of aqueous effluents containing methyl tert-butyl ether and/or methyl tert-amyl ether by Mycobacterium austroafricanum CNCM I-2562

(30) Priorité: 15.10.2001 FR 0113299
(43) Date de publication de la demande: 16.04.2003
(73) Titulaire: Institut Français du Pétrole, 92852 Rueil-Malmaison Cedex (FR)
(72) Inventeur: Fayolle, Francoise, 92140 Clamart (FR); Francois, Alan, 95230 Soisy s/Montmorency (FR); Monot, Frédéric, 92000 Nanterre (FR)

(56) Documents cités:
- WO-A-00/63343
- WO-A-01/34528
- US-A- 5 814 514
- HANSON J R ET AL: "Biodegradation of Methyl tert-Butyl Ether by a Bacterial Pure Culture", APPLIED AND ENVIRONMENTAL MICROBIOLOGY, AMERICAN SOCIETY FOR MICROBIOLOGY, US, vol. 65, no. 11, 1 November 1999 (1999-11-01), pages 4788-4792, XP002965397, ISSN: 0099-2240

## Description

L'invention décrit des microrganismes capables de dégrader le méthyl-*tert*-butyl éther et/ou le tertioamylmethyl éther.

Elle s'applique particulièrement à l'industrie du traitement de l'eau.

Il est connu que le méthyl-*tert*-butyl éther ou le méthyl-tert-amyl éther -désigné respectivement ci-après sous le terme de MTBE et TAME- est un des éthers qui peut être utilisé comme additif oxygéné dans les essences sans plomb dans le but d'augmenter leur indice d'octane. L'utilisation croissante d'additifs comme le MTBE ou le TAME entraine des volumes importants stockés et transportés, en mélange dans les essences notamment. Il est donc nécessaire de connaitre le devenir de ce composé en cas de déversement accidentel conduisant à une pollution des sols et des eaux souterraines ou de surface. Le MTBE ou le TAME est un éther produit par condensation du méthanol respectivement sur l'isobutène ou l'isopentène. Sa structure, qui comporte une liaison éther ainsi qu'un carbone tertiaire, est de nature à le rendre résistant à la biodégradation par les microorganismes présents dans l'environnement.

La littérature récente relative à la biodégradation des éthers d'alkyle utilisés dans les essences indique que le métabolisme de ces composés dans l'environnement n'est pas un phénomène commun et qu'il est relativement lent, tant en conditions aérobies qu'anaérobies.

L'art antérieur est illustré par le brevet WO-0134528 et par les brevets suivants :
- le brevet US-A--5.814,514 décrit l'utilisation de souches bactériennes utilisant le propane ou l'isopropanol comme substrat carboné de croissance (celui-ci étant oxydé), les éthers étant dégradés par co-métabolisme. Dans ce brevet le propane (ou l'isopropanol est introduit par voie externe dans le milieu avec l'éther à dégrader,
- le brevet WO-00/63 343 enseigne une culture pure de Rhodococcus (culture A) capable de dégrader le MTBE utilisé comme source carbonée de croissance. Le tableau et la figure présentés à la page 22 du brevet montrent les degrés d'homologie avec d'autres souches classées par ordre décroissant. La "culture A" a des taux de similitude forts avec des souches de Rhodococcus et plus faibles avec les souches de Mycobacterium citées. Les souches de Mycobacterium sont ici mentionnées à titre de comparaison pour montrer justement que la "culture A" est un Rhodococcus et non un Mycobacterium.

La publication Hanson J.R. et al, "Biodegradation of Methyl tert-Butyl Ether by a Bacterial Pure Culture", Applied and Environmental Microbiology, vol. 65, no. 11, 1 novembre 1999, pages 4788-4792 décrit une souche de bactéries PM1 capable de pousser sur du MTBE comme seule source de carbone.

La Demanderesse avait isolé et décrit dans le brevet FR-A-2.787.783 une bactérie, *Gordonia terrae* I-2194, pour sa capacité à pousser sur ETBE comme source de carbone et d'énergie. Cette bactérie dégradait l'ETBE en tertio-butanol (TBA). Cette bactérie s'était également avérée capable de dégrader le MTBE en présence d'un substrat de croissance carboné. Néanmoins, que le composé à dégrader soit l'ETBE ou le MTBE, du TBA était formé dans le milieu de croissance. Une deuxième bactérie devait donc être mise en oeuvre pour une épuration totale des effluents à traiter. Trois bactéries différentes ont été isolées et sont capables de pousser sur TBA comme source de carbone et d'énergie. Il s'agit selon le brevet FR-A-2.800.748 de P.*cepacia* I-2052, *Alcaligenes austroafricanum* I-2561 et *Mycobacterium austroafricanum* I-2562.

Il en ressort qu'il est nécessaire de trouver et d'identifier de nouveaux microorganismes capables de biodégrader le MTBE ou le TAME et d'étudier leur mise en oeuvre dans des procédés de traitement de l'eau permettant d'abaisser significativement les concentrations résiduelles en MTBE ou en TAME d'eaux résiduaires urbaines ou industrielles ou de nappes aquifères contaminées, désignées sous le nom général d'effluents, contaminées par ce produit.

Une des nouvelles bactéries isolée par la Demanderesse et permettant de dégrader le tertio-butanol (TBA) en solution dans l'eau, *Mycobacterium austroafricanum* CNCM I-2562, s'est avérée capable de dégrader également le MTBE en solution dans l'eau. Un fait notable est que *Mycobacterium austroafricanum* CNCM I-2562 est capable d'utiliser le MTBE comme seule source de carbone et d'énergie.

Un des objectifs de l'invention est de décrire les nouvelles potentialités de cette bactérie capable de dégrader le MTBE ou le TAME contenu en solution dans l'eau, afin qu'elle soit utilisée pour le traitement des eaux polluées.

De manière plus détaillée, l'invention décrit une souche bactérienne, isolée de l'environnement, qui est capable de dégrader le MTBE et/ou le TAME de façon complète sans accumulation d'intermédiaire de dégradation. Cette nouvelle bactérie déposée à l'Institut Pasteur (CNCM de l'Institut Pasteur, 25, rue du Docteur Roux, F-75724 PARIS CEDEX 15) est *Mycobacterium sp.* I-2562, identifiée ultérieurement comme étant Mycobacterium austroafricanum CNCM I-2562.

Plus précisément, l'invention concerne un procédé de traitement d'effluents aqueux contenant au moins un éther du groupe méthyl *tertio* butyl éther (MTBE) et du méthyl tertio amyl éther (TAME) afin de réduire la concentration dudit éther, caractérisé en ce qu'on fait croître en conditions aérobies une bactérie *Mycobacterium austroafricanum* CNCM I-2562 en présence d'un substrat de croissance contenant le dit éther comme seule source de carbone et d'énergie et on fait dégrader le dit éther par ladite bactérie jusqu'aux produits ultimes de dégradation, le dioxyde de carbone et l'eau.

Les produits intermédiaires de dégradation du MTBE et du TAME, respectivement en particulier le tertio-butanol (TBA) et le tertio-amylalcool (TAA) sont aussi totalement minéralisés.

Selon une caractéristique de l'invention, les effluents aqueux peuvent être un aquifère contenant au moins du MTBE et/ou du TAME et dans lequel on introduit sous la forme adéquate la bactérie *Mycobacterium austroafricanum* CNCM I-2562 afin de réduire la concentration de MTBE et/ou de TAME dans l'aquifère.

Selon une autre caractéristique, on peut ajouter au substrat de croissance de l'extrait de levure dans des proportions comprises entre 10 et 200 mg/L.

La bactérie a été isolée de boues activées collectées à une station d'épuration d'eaux résiduaires urbaines, qui ont été traitées selon des techniques d'enrichissement en microrganismes spécifiques.

La souche bactérienne résultante a été isolée après enrichissement spécifique sur *tertio*-butanol (TBA) ou tertio-amylalcool et testée en culture pure pour sa capacité à dégrader le MTBE ou le TAME.

Dans ces expérimentations, il faut noter que le MTBE ou le TAME peut être fourni comme seule source de carbone mais des additifs nutritionnels peuvent être rajoutés au milieu de croissance pour accélérer la croissance sur MTBE ou sur TAME de cette nouvelle bactérie. Des concentrations en MTBE et/ou en TAME élevées, par exemple au plus égales à 2 g/L sont dégradées par la nouvelle bactérie à des taux pouvant aller jusqu'à 100%. *Mycobacterium austroafricanum* CNCM I-2562 est également capable de dégrader des concentrations très faibles en MTBE ou en TAME. On a obtenu d'excellents résultats avec des concentrations en MTBE ou en TAME dans l'effluent aqueux comprises entre 0,05 mg/L et 200 mg/L.

La mise en oeuvre de ces bactéries pour le traitement en continu d'effluents pollués par du MTBE et/ou du TAME peut être réalisée, par exemple dans un biofiltre où les bactéries sont fixées sur un support minéral ou organique ou bien elles peuvent être ajoutées comme inoculum à des boues de station d'épuration, ou dans tout autre système adapté au traitement des eaux et des sols (biobarrière). Plus précisément, on peut faire développer la bactérie sur un système de biofiltre ou de biobarrière de volume adéquat, on introduit les effluents contenant ledit éther en présence d'air ou d'oxygène dans le biofiltre ou dans la biobarrière à un débit d'alimentation en MTBE et/ou en TAME inférieur à 30 mg/L de biofiltre et par heure et on soutire l'effluent avec une concentration réduite en ledit éther.

L'invention sera mieux comprise par les exemples suivants donnés à titre illustratif ainsi que par les figures, parmi lesquelles :
- La figure 1 illustre la croissance de Mycobacterium austroafricanum CNCM I-2562 sur un milieu minéral en présence de MTBE comme seule source de carbone.
- La figure 2 représente l'effet de l'addition d'extrait de levure sur la croissance de Mycobacterium austroafricanum CNCM I-2562 sur MTBE comme seule source de carbone.
- La figure 3 montre la croissance de Mycobacterium austroafricanum CNCM I-2562 sur différentes concentrations élevées en MTBE en présence d'extrait de levure (100 mg/L).
- La figure 4 illustre la croissance de la souche sur TAME comme seule souche de carbone.

### EXEMPLE 1

### Croissance de Mycobacterium austroafricanum CNCM I-2562 sur un milieu minéral en présence de MTBE comme seule source de carbone

On effectue une préculture de la bactérie *Mycobacterium austroafricanum* CNCM I-2562: on ensemence la souche *Mycobacterium austroafricanum* CNCM I-2562 sur milieu minéral salin MM supplémenté en *tertio*-butanol ou TBA à 1 g/L comme source de carbone et d'énergie. Le milieu MM a la composition suivante :
- KH₂PO₄ 1,4 g
- K₂HPO₄ 1,7 g
- NaNO₃ 1,5 g
- MgSO₄, 7H₂O 0,5g
- CaCl₂, 2H₂O 0,04 g
- FeCl₃, 6H₂O 0,012 g
- Solution concentrée de vitamines 1 mL
- Solution concentrée d'oligoéléments 1 mL
- H₂O q.s.p.1 litre

La solution concentrée de vitamines a la composition suivante pour 1 litre d'eau distillée :
- Biotine 200 mg
- Riboflavine 50 mg
- Acide nicotinamique 50 mg
- Panthoténate 50 mg
- Acide p-aminobenzoïque 50 mg
- Acide folique 20 mg
- Thiamine 15 mg
- Cyanocobalamine 1,5 mg

La solution concentrée d'oligoéléments a la composition suivante pour 1 litre d'eau distillée :
- CuSO₄, 5 H₂O 0,1 g
- MnSO₄, 2H₂O 1 g
- ZnSO₄, 7 H2O 1 g
- AlCl₃, 6H₂O 0,4 g
- NiCl₂,6 H₂O 0,25 g
- H₃BO₃ 0,1 g
- CoCl₂, 6 H₂O 1 g
- Na₂MoO₄, 2H₂O 1 g
- Na₂WO₄, 2H₂O 1 g

Après croissance, cette préculture est centrifugée, lavée avec du milieu MM pour enlever toute trace de TBA résiduel et le culot bactérien ainsi obtenu est utilisé pour ensemencer 50 mL de milieu MM auquel on ajoute du MTBE à une concentration finale d'environ 70 mg/L dans une fiole d'Erlenmeyer d'une contenance de 500 mL fermé avec un bouchon téflonné afin d'éviter toute perte de MTBE au cours de la croissance. Un prélèvement est effectué au temps t=0 pour une mesure de la densité optique à 600 nm (D. O._{600 nm}) et un dosage du MTBE au départ par analyse en chromatographie en phase gazeuse (CPG). La fiole est ensuite incubée à 30°C dans un agitateur rotatif. Un prélèvement pour la mesure de la D.O._{600 nm} ainsi qu'un dosage du substrat et de ses éventuels produits de dégradation est effectué à intervalle régulier. Lorsque tout le MTBE de départ a été consommé, une deuxième addition de MTBE est réalisée à t = 80 heures et seul le suivi de la mesure de la DO_{600 nm} est alors effectué dans cette deuxième partie de l'expérimentation.

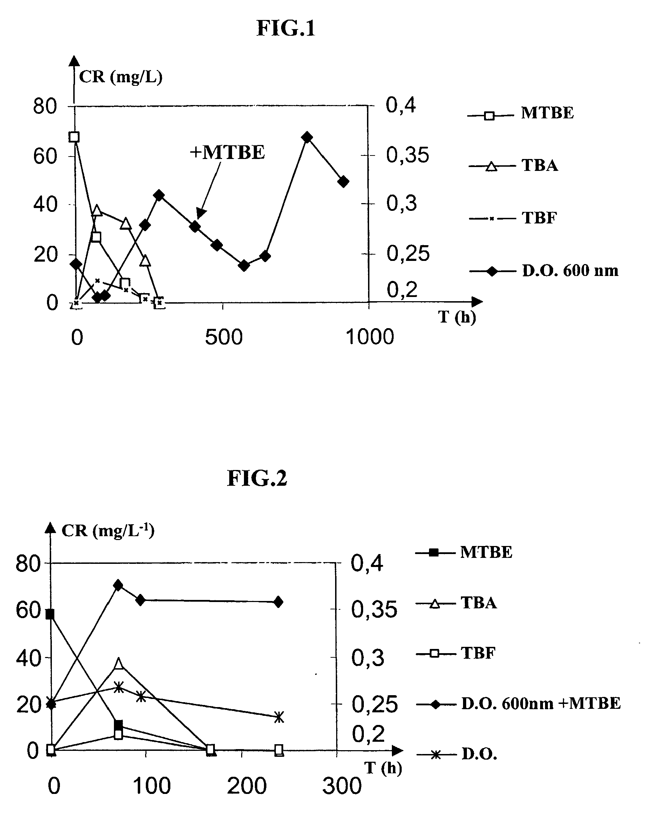

Le résultat de cette expérimentation est présenté dans la figure 1 qui montre en ordonnée la concentration résiduelle en mg/L de trois constituants (MTBE, TBA, TBF) ainsi que la densité optique en fonction du temps, en abscisse.

Comme on le voit sur cette figure, la première addition de MTBE se traduit par une croissance de la bactérie *Mycobacterium austroafricanum* CNCM I-2562. Le MTBE est dégradé en partie en *tertio*-butyl formiate (TBF) et en *tertio*-butyl alcool (TBA). Ces deux composés sont ensuite utilisés comme substrat de croissance et on obtient une augmentation de la DO pendant leur consommation. Quand il ne reste plus de composé carboné, la DO baisse légèrement, ceci est classiquement observé en fin de croissance des bactéries. Une deuxième addition de MTBE permet alors la reprise de la croissance. Cet exemple montre bien que *Mycobacterium austroafricanum* CNCM I-2562 utilise le MTBE comme seule source de carbone et d'énergie. Aucun autre substrat carboné n'est nécessaire. Les produits intermédiaires accumulés sont ensuite eux-mêmes dégradés. La minéralisation du MTBE peut donc avoir lieu sans présence d'un autre substrat carboné d'origine externe et en absence d'une autre souche dégradant le TBA et le TBF.

### EXEMPLE 2

### Effet de l'addition d'extrait de levure sur la croissance de Mycobacterium austroafricanum CNCM I-2562 sur MTBE comme seule source de carbone

On a voulu tester si l'addition d'une faible quantité d'extrait de levure dans le milieu de culture pouvait permettre une utilisation plus rapide du MTBE fourni comme seule source de carbone à *Mycobacterium austroafricanum* CNCM I-2562.

Une préculture de la bactérie *Mycobacterium austroafricanum* CNCM I-2562 est réalisée dans les mêmes conditions que celles décrites dans l'exemple 1. On prépare 50 mL de milieu MM contenant de l'extrait de levure à une concentration finale de 100 mg/L dans une fiole d'Erlenmeyer à bouchon vissé et téflonné et on ajoute du MTBE à une concentration similaire à celle décrite dans l'exemple 1. Comme décrit dans l'exemple 1, après ensemencement du milieu de culture on suit la croissance et le devenir du substrat. Le résultat de cette expérimentation est présenté sur la figure 2 qui montre, en ordonnée, la concentration résiduelle en mg/L des trois constituants (MTBE, TBA, TBF) ainsi que la densité optique avec ou sans MTBE, en fonction du temps en abscisse.

Comme on le voit sur cette figure, et par comparaison avec le résultat de l'exemple 1, l'addition d'une faible quantité d'extrait de levure permet d'augmenter la vitesse de dégradation du MTBE. Alors que dans l'exemple précédent, une durée d'incubation de la culture de 288 heures était nécessaire pour dégrader totalement le MTBE (c'est-à-dire, le MTBE lui-même et ses intermédiaires de dégradation que sont le TBF et le TBA), une durée bien moindre, égale à 168 heures, est nécessaire en présence d'extrait de levure pour obtenir le même résultat. On notera que, dans le témoin de l'expérimentation qui est constitué par une fiole de milieu MM supplémenté avec de l'extrait de levure à la même concentration que dans l'essai (100 mg.L) ensemencée avec la souche *Mycobacterium austroafricanum* CNCM I-2562 mais sans addition de MTBE, aucune croissance n'est observée. Ce qui prouve bien que la croissance de la bactérie *Mycobacterium austroafricanum* CNCM I-2562 est bien exclusivement due à sa capacité de croissance sur MTBE.

### EXEMPLE 3

### Croissance de Mycobacterium austroafricanum CNCM I-2562 sur différentes concentrations élevées en MTBE en présence d'extrait de levure (100 mg.L⁻¹)

On a voulu tester les capacités de croissance de la bactérie *Mycobacterium austroafricanum* CNCM I-2562 sur des concentrations plus élevées de MTBE. L'expérimentation est la même que celle décrite dans l'exemple 2 , c'est-à-dire effectuée sur du milieu MM supplémenté avec de l'extrait de levure à 100 mg/L. On ensemence trois fioles d'Erlenmeyer de 1 L à bouchon téflonné et vissé contenant 100 mL de milieu MM supplémenté en extrait de levure avec une préculture préparée comme dans l'exemple 1. On ajoute à deux de ces fioles du MTBE à 200 ou à 400 mg/L. La troisième de ces fioles ne contient pas de MTBE et est le témoin de l'expérimentation sur extrait de levure seul. On effectue un prélèvement pour mesurer la DO_{600 nm} initiale.

Ces cultures sont incubées à 30°C sur un agitateur rotatif. Des prélèvements sont effectuées régulièrement pour le suivi de la croissance par mesure de la DO_{600 nm}.

Du MTBE est ensuite ajouté à intervalles réguliers au milieu de culture à raison de 100 mg/L.

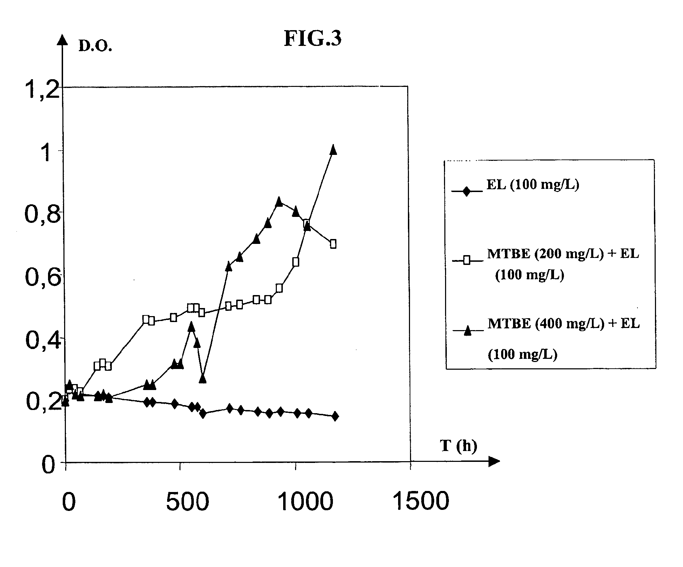

Le résultat de cette expérimentation est montré dans la figure 3 qui représente en ordonnée la densité optique à 600 nm à différentes concentrations de MTBE en présence d'extrait de levure en fonction du temps en heures, en abscisse ainsi que la densité optique en présence d'extrait de levure seul. Sur cette figure, les additions successives de MTBE à 100 mg/L sont figurées par des flêches. Cette courbe montre que la croissance est bien liée à l'addition de MTBE dans le milieu de culture.

Sur cette figure, on voit que l'addition de concentrations plus élevées se traduit par un temps de latence avant croissance qui est important. Il est d'une centaine d'heures à une concentration en MTBE de 200 mg/L, et d'environ 200 heures à une concentration en MTBE de 400 mg/L. Par ailleurs, il est notable que la souche *Mycobacterium austroafricanum* CNCM I-2562 est capable de se développer même à des concentrations élevées de MTBE. Comme dans l'exemple 2, on n'observe aucune croissance sur extrait de levure seul.

### EXEMPLE 4

### Croissance de Mycobacterium austroafricanum CNCM I-2562 sur un milieu minéral en présence de TAME comme seule source de carbone

On effectue une préculture de la bactérie *Mycobacterium austroafricanum* CNCM I-2562: on ensemence la souche *Mycobacterium austroafricanum* CNCM I-2562 sur milieu minéral salin MM2 supplémenté en tertio-butanol ou TBA à 1 g.L⁻¹ comme source de carbone et d'énergie. Le milieu MM2 a la composition suivante :
- KH₂PO₄ 1,4 g
- K2HPO₄ 1,7 g
- (NH₄)₂SO₄ 1,2 g
- MgSO₄, 7H₂O 0,5g
- CaCl₂, 2H₂O 0,04 g
- FeCl₃, 6H₂O 0,012 g
- Extrait de levure 0,1 g
- Solution concentrée de vitamines 1 mL
- Solution concentrée d'oligoéléments 1 mL
- H₂O q.s.p.1 litre

La solution concentrée de vitamines a la composition suivante pour 1 litre d'eau distillée.:
- Biotine 200 mg
- Riboflavine 50 mg
- Acide nicotinamique 50 mg
- Panthoténate 50 mg
- Acide p-aminobenzoïque 50 mg
- Acide folique 20 mg
- Thiamine 15 mg
- Cyanocobalamine 1,5 mg

La solution concentrée d'oligoéléments a la composition suivante pour 1 litre d'eau distillée :
- CuSO₄, 5 H₂O 0,1 g
- MnSO₄, 2H₂O 1 g
- ZnSO₄, 7 H2O 1 g
- AlCl₃, 6H₂O 0,4 g
- NiCl₂,6 H₂O 0,25 g
- H₃BO₃ 0,1 g
- CoCl₂, 6 H₂O 1 g
- Na₂MoO₄, 2H₂O 1 g
- Na₂WO₄, 2H₂O₂ 1 g

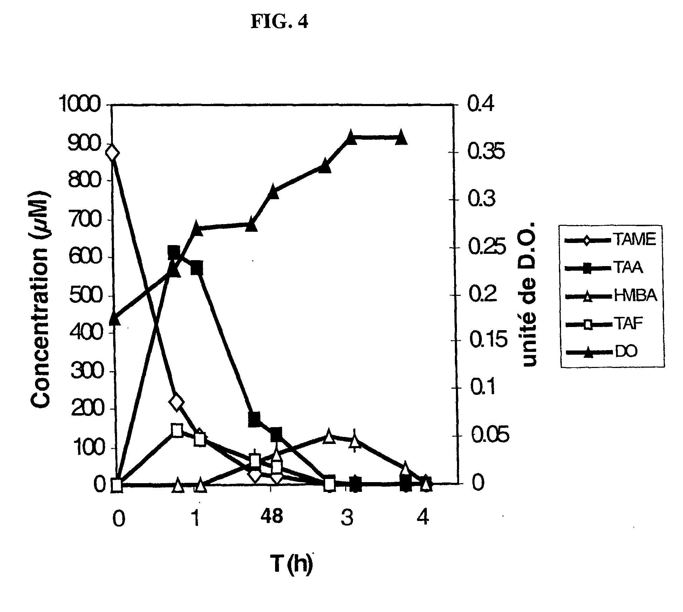

Après croissance, cette préculture est centrifugée, lavée avec du milieu MM2 pour enlever toute trace de TBA résiduel et le culot bactérien ainsi obtenu est utilisé pour ensemencer 3 fioles de 100 mL de milieu MM2 auxquelles on ajoute du TAME à une concentration finale d'environ 90 mg.L⁻¹ dans des fioles d'Erlenmeyer d'une contenance de 500 mL fermées avec des bouchons téflonnés afin d'éviter toute perte de TAME au cours de la croissance. Un prélèvement est effectué au temps t=0 pour une mesure de la densité optique à 600 nm (D. O. _{600 nm}) et un dosage du TAME au départ par analyse en chromatographie en phase gazeuse (CPG). Les fioles sont ensuite incubées à 30°C dans un agitateur rotatif. Un prélèvement pour la mesure de la D.O._{600 nm} ainsi qu'un dosage du substrat et de ses éventuels produits de dégradation sont effectués à intervalle régulier.
Le résultat de cette expérimentation est présenté dans la figure 4 qui montre en ordonnée une moyenne des concentrations résiduelles en µM (micromoles) du TAME et de trois de ces intermédiaires de dégradation (le *tert*-amyl formiate ou TAF, le *tert-*amyl alcool ou TAA et l'acide hydroxyméthylbutyrique ou HMBA) ainsi que la densité optique en fonction du temps, en abscisse, exprimé en heures.

Comme on le voit sur cette figure, le TAME est dégradé en partie en *tertio-*amyl formiate (TAF) et en *tertio-*amyl alcool (TAA). Ces deux composés sont ensuite utilisés comme substrat de croissance et on peut mettre en évidence la présence de l'acide hydroxyméthylbutyrique (HMBA) qui est un intermédiaire de la dégradation du TAA. On mesure une augmentation de la DO pendant la consommation du TAME et de ses métabolites. Aucun autre substrat carboné n'est nécessaire. Les produits intermédiaires accumulés sont ensuite eux-même dégradés. La minéralisation du TAME peut donc avoir lieu sans présence d'un autre substrat carboné et en absence d'une autre souche dégradant le TAF, le TAA et l'HMBA.

## Revendications

1. Procédé de traitement d'effluents aqueux contenant au moins un éther du groupe le méthyl *tertio* butyl éther (MTBE) et du méthyl-tertio amyl éther (TAME) afin de réduire la concentration dudit éther, **caractérisé en ce qu'**on fait croître en conditions aérobies une bactérie *Mycobacterium austroafricanum* CNCM I-2562 en présence d'un substrat de croissance contenant le dit éther comme seule source de carbone et d'énergie et on fait dégrader le dit éther par ladite bactérie jusqu'aux produits ultimes de dégradation, le dioxyde de carbone et l'eau.

2. Procédé selon la revendication 1 dans lequel les effluents aqueux sont un aquifère contenant au moins du MTBE et/ou du TAME et dans lequel on introduit sous la forme adéquate la bactérie *Mycobacterium austroafricanum* CNCM I-2562 afin de réduire la concentration de MTBE et/ou de TAME dans l'aquifère.

3. Procédé selon l'une des revendications 1 et 2 dans lequel on ajoute au substrat de croissance de l'extrait de levure, dans des proportions comprises entre 10 et 200 mg/L.

4. Procédé selon d'une des revendications 1 à 3 dans lequel la concentration en MTBE et/ou en TAME dans les effluents aqueux à traiter est au plus égale à 2 g/L et de préférence comprise entre 0,05 mg/L et 200 mg/L.

5. Procédé selon l'une des revendications 1 à 4 dans lequel on fait développer la dite bactérie sur un système de biofiltre ou de biobarrière de volume adéquat, on introduit les effluents contenant le dit éther en présence d'air ou d'oxygène dans le biofiltre ou la biobarrière à un débit d'alimentation en MTBE et/ou en TAME inférieur à 30 mg/L de biofiltre et par heure et on soutire l'effluent avec une concentration réduite en le dit éther.

## Claims

1. Process for treatment of aqueous effluents that contain at least one ether of the methyl-tert-butyl ether (MTBE) group and the methyl-tert-amyl ether (TAME group so as to reduce the concentration of said ether, **characterized in that** under aerobic condition, a Mycobacterium austroafricanum CNCM I-2562 bacterium is grown in the presence of a growth substrate that contains said ether as a sole source of carbon and energy, and said ether is degraded by said bacterium down to the final degradation products, carbon dioxide and water.

2. Process according to claim 1, wherein the aqueous effluents are an aquifer that contains at least MTBE and/or TAME and in which the Mycobacterium austroafricanum CNCM 1-2562 bacterium is introduced in a suitable form so as to reduce the concentration of MTBE and/or TAME in the aquifer.

3. Process according to one of claims 1 and 2, wherein yeast extract, in proportions of between 10 and 200 mg/L, is added to the growth substrate.

4. Process according to one of claims 1 to 3, wherein the concentration of MTBE and/or TAME in the aqueous effluents that are to be treated is at most equal to 2 g/L and preferably between 0.05 mg/L and 200 mg/L.

5. Process according to one of claims 1 to 4, wherein said bacterium is developed in a biofilter system or a biobarrier system with a suitable volume; the effluents that contain said ether in the presence of air or oxygen are introduced into the biofilter or the biobarrier at a feed flow of MTBE and/or TAME that is less than 30 mg/L of biofilter and per hour and the effluent is drawn off with a reduced concentration of said ether.

## Patentansprüche

1. Verfahren zur Behandlung wässriger Abflüsse, die wenigstens einen Ether der Gruppe Methyl-tertiär-butyl-ether (MTBE) und Tertiär-amylmethyl-ether (TAME) enthalten, um die Konzentration des Ethers zu verringern, **dadurch gekennzeichnet, dass** man unter aeroben Bedingungen ein Bakterium *Mycobacterium austroafricanum* CNCM I-2562 in Gegenwart eines Wachstumssubstrats, das den Ether als einzige Kohlenstoff- und Energiequelle enthält, wachsen lässt und den Ether durch das Bakterium bis zu den endgültigen Abbauprodukten Kohlendioxid und Wasser abbauen lässt.

2. Verfahren nach Anspruch 1, wobei die wässrigen Abflüsse ein Grundwasserspeicher sind, der wenigstens MTBE und/oder TAME enthält und in den in geeigneter Form das Bakterium *Mycobacterium austroafricanum* CNCM I-2562 zum Verringern der Konzentration von MTBE und/oder TAME im Grundwasserspeicher eingeführt wird.

3. Verfahren nach einem der Ansprüche 1 und 2, wobei dem Wachstumssubstrat Hefeextrakt in Anteilen zwischen 10 und 200 mg/l zugefügt wird.

4. Verfahren nach einem der Ansprüche 1 bis 3, wobei die Konzentration von MTBE und/oder TAME in den zu behandelnden wässrigen Abflüssen höchstens 2 g/l entspricht und vorzugsweise zwischen 0,05 mg/l und 200 mg/l.

5. Verfahren nach einem der Ansprüche 1 bis 4, wobei das Bakterium auf einem Biofilter- oder Biobarrierensystem geeigneten Volumens entwickelt wird, die den Ether enthaltenden Abflüsse in Gegenwart von Luft oder von Sauerstoff in den Biofilter oder die Biobarriere bei einem Einspeisungsdurchsatz von MTBE und/oder TAME von weniger als 30 mg/l Biofilter und pro Stunde eingeleitet werden und der Abfluss mit einer verringerten Konzentration des Ethers abgezogen wird.
